# FASCICULE DE BREVET EUROPEEN

(11) **EP 3 200 675 B1**
(45) Date de publication et mention de la délivrance du brevet: **17.02.2021**
(21) Numéro de dépôt: 15771665.5
(22) Date de dépôt: 02.10.2015
(51) Int. Cl.: A61B 5/00, A61F 2/82

(54) **DISPOSITIF MEDICAL MUNI DE CAPTEURS A IMPEDANCE VARIABLE**
MIT SENSOREN MIT VARIABLER IMPEDANZ AUSGESTATTETE, MEDIZINISCHE VORRICHTUNG
MEDICAL DEVICE PROVIDED WITH SENSORS HAVING VARIABLE IMPEDANCE

(30) Priorité: 03.10.2014 FR 1459531
(43) Date de publication de la demande: 09.08.2017
(73) Titulaire: Centre National de la Recherche Scientifique, 75794 Paris Cedex 16 (FR); Ecole Polytechnique, 91128 Palaiseau Cedex (FR); Ecole Normale Supérieure de Cachan, 94230 Cachan (FR)
(72) Inventeur: BARAKAT, Abdul, F-91190 Gif-sur-Yvette (FR); BOZSAK, Franz, F-92340 Bourg-la-Reine (FR); BONNASSIEUX, Yvan Eric, F-75013 Paris (FR); LE PIOUFLE, Bruno, F-75013 Paris (FR); FRANCAIS, Olivier, F-77000 Melun (FR); CARREEL, Bruno, F-75014 Paris (FR)
(74) Mandataire: Icosa
(86) Numéro de dépôt international: PCT/EP2015/072859
(87) Numéro de publication internationale: WO 2016/050972

(56) Documents cités:
- WO-A1-01/37726
- WO-A1-99/42176
- US-A1- 2002 177 782
- US-A1- 2011 251 469
- US-B1- 6 206 835

## Description

La présente invention concerne un dispositif médical implantable dans le corps humain muni de capteurs, à impédance variable.

L'invention vise également un système médical comprenant un tel dispositif médical et un procédé d'interrogation d'un tel dispositif médical, notamment dans un système médical.

L'invention vise notamment un dispositif médical implantable tel un stent (parfois également appelé « endoprothèse artérielle », « tuteur vasculaire » voire même simplement « ressort ») muni de capteurs.

Un stent est un dispositif de forme tubulaire réalisé par un maillage déformable, notamment en métal ou en un matériau polymère biodégradable. Le stent est introduit dans le corps d'un patient dans un état replié, mailles fermées, puis est étendu à l'intérieur du corps du patient, par exemple par angioplastie qui provoque le déploiement des mailles. Le stent, déployé, permet de maintenir ouverte une cavité dans le corps du patient. Il est connu que la mise en place d'un stent peut notamment provoquer une inflammation des tissus, une hyperplasie et/ou une coagulation de sang.

Par conséquent, un stent peut être muni de capteurs, qui permettent de surveiller l'état des tissus autour du stent pour, éventuellement, adapter le traitement du patient en conséquence. Des capteurs peuvent également être prévus pour s'assurer que le stent remplit sa fonction de maintien d'une cavité ouverte.

Il est connu d'interroger un stent, c'est-à-dire de recueillir une information provenant de ce stent, à l'aide d'un dispositif d'interrogation sans contact, conservé à l'extérieur du patient. Généralement, un tel dispositif d'interrogation sans contact est conformé pour mesurer un champ électromagnétique émis par le stent implanté dans le patient.

Le brevet EP-B-2 271 933 décrit ainsi un procédé pour caractériser des cellules au voisinage d'un dispositif médical implanté dans un patient, notamment un stent, par des mesures d'impédance à différentes fréquences.

La demande WO-A-2009/1 361 677 décrit un dispositif médical implantable tel qu'un stent, présentant une surface conductrice d'électricité et un capteur d'impédance pour mesurer l'impédance de la surface conductrice du dispositif médical implantable, à différentes fréquences, en utilisant la surface conductrice comme électrode. Les mesures effectuées sont utilisées pour déterminer le degré de resténose des tissus au niveau du dispositif implantable, c'est-à-dire l'épaisseur de tissu ayant poussé au niveau de la surface conductrice du dispositif médical implantable.

Ces documents enseignent des méthodes donnant une information globale sur le dispositif implantable, sans permettre d'obtenir indépendamment les mesures réalisées par chaque capteur dont est muni le dispositif médical implantable.

Par ailleurs, il est connu de US-B-8 478 378, un stent muni de capteurs répartis sur sa surface interne, orientée vers le passage à travers le stent, ou surface « luminale ». Les capteurs sont configurés pour envoyer un signal de sortie caractéristique propre en réponse à une excitation. Le signal caractéristique propre peut notamment être une longueur d'onde propre à chacun des capteurs. US-B-8 478 378 indique qu'ainsi un signal de sortie incluant des signaux de tous ou de la plupart des capteurs suggère qu'un grand nombre de capteurs ne sont pas recouverts d'une couche de cellules endothéliales.

Enfin, la demande WO-A-2011/121581 décrit un dispositif médical implantable capable de répondre à un champ électromagnétique d'interrogation émis par un dispositif d'interrogation distant. Le dispositif médical implantable est muni d'une pluralité de modulateurs constitués par des puces RFID (de l'anglais « Radio-Frequency IDentification » ou radio-identification). Les puces RFID sont adaptées pour que le dispositif médical implantable réponde à un champ électromagnétique d'interrogation selon une modulation générant un code d'identification respectif unique.

L'utilisation de puces RFID comme capteurs dans le dispositif médical limite cependant le nombre de capteurs dont il peut être muni. La multiplication des puces RFID augmente en effet d'autant le prix du dispositif médical. Ces puces RFID ne peuvent, en outre, être utilisées que comme capteurs d'impédance. Par ailleurs, selon ce document, le dispositif médical doit être au moins en partie en un matériau métallique ayant une bonne conduction électrique. Enfin, selon ce document, les puces RFID doivent être implantée dans la structure même du dispositif médical implantable, rendant la réalisation de celui-ci particulièrement complexe.

Il est également connu de WO-A-01/37 726 ou US-B-6 206 835, des dispositifs médicaux implantables. Ces dispositifs médicaux comportent une structure implantable dans le corps pour assister la réalisation d'une fonction vitale dans le corps. Un ou plusieurs capteurs sont associés à cette structure implantable, qui permet(tent) de mesurer un paramètre associé à la structure. Enfin, ces dispositifs médicaux comportent un circuit de communication couplé au(x) capteur(s) pour délivrer un signal fonction du paramètre mesuré et pour transmettre ce signal à un dispositif de réception, à l'extérieur du corps, de manière non invasive.

L'invention a pour objectif de pallier les problèmes évoqués plus haut. Notamment, l'invention a pour objectif de proposer un dispositif médical de structure simple et donc de coût limité, permettant de distinguer les grandeurs mesurées par différents capteurs dont est muni le dispositif médical. Le dispositif médical est implantable dans le corps du patient et configuré pour permettre de déterminer, sans intrusion dans le corps du patient, s'il est correctement implanté.

L'invention propose un dispositif médical comprenant un circuit électrique de mesure, dans lequel sont branchés au moins deux capteurs à impédance variable en fonction d'une grandeur physique captée, une source d'énergie électrique pour alimenter le circuit électrique de mesure, une antenne pour émettre un champ électromagnétique fonction de l'impédance du circuit électrique de mesure, chacun des capteurs étant associé à un interrupteur pour court-circuiter le capteur dans ledit circuit de mesure, le dispositif médical comprenant en outre un système de commande des interrupteurs pour successivement commander l'ouverture ou la fermeture des interrupteurs les uns après les autres, selon des configurations déterminées et dans lequel le système de commande des interrupteurs comporte un circuit de commande, alimenté par la source d'énergie électrique, le système de commande étant conformé pour normalement maintenir fermés les interrupteurs et pour les ouvrir successivement puis pour les refermer de sorte qu'à chaque instant un unique interrupteur est ouvert.

Ainsi, selon l'invention, le dispositif médical est muni de tout type de capteurs à impédance variable reliés ensemble dans un circuit dit de mesure. Un système de commande permet de court-circuiter les différents capteurs selon des configurations prédéterminées, de sorte que le champ électromagnétique émis par le dispositif médical correspond à la configuration du circuit de mesure. En réalisant des mesures successives, correspondant à des configurations linéairement indépendantes il est très facilement possible d'obtenir une information qualitative sur les valeurs mesurées par chacun des capteurs du dispositif médical, disposés à des emplacements connus sur le dispositif médical.

Par « court-circuiter un capteur », on entend créer une configuration du circuit telle que le courant traversant le capteur est nul, les autres capteurs pouvant être alimenté en courant. En d'autres termes, « court-circuiter un capteur » signifie ici couper l'alimentation en courant de ce capteur.

De préférence, le dispositif médical comporte une ou plusieurs des caractéristiques suivantes, prises seules ou en combinaison :
- chaque interrupteur est formé par un ou des transistors, notamment un ou des transistors à effet de champs FET, plus particulièrement un ou des transistors à effet de champs à grille métal-oxyde MOS-FET, à enrichissement ou à appauvrissement, à canal N ou à canal P, un ou des MEMS, ou un ou des interrupteurs mécaniques ;
- le système de commande comporte des composants implantés directement dans le circuit de mesure, de préférence pour commander successivement l'ouverture ou la fermeture des différents interrupteurs, les uns après les autres ;
- chaque ensemble d'un interrupteur et d'un capteur est monté en série et les ensembles d'un interrupteur et d'un capteur sont montés en parallèle les uns par rapport aux autres ;
- chaque ensemble d'un interrupteur et d'un capteur est monté en parallèle et les ensembles d'un interrupteur et d'un capteur sont montés en série les uns des autres ;
- la source d'énergie électrique comporte une surface conductrice de courant du dispositif médical, adaptée à induire un courant électrique sous l'effet d'un champ électromagnétique ;
- au moins un des capteurs est disposé sur une surface du dispositif médical, destinée à être en contact avec une partie du corps sur laquelle le dispositif est appliqué ou dans laquelle le dispositif est implantable ;
- l'antenne est formée par une partie au moins du dispositif médical ;
- le circuit de mesure comporte une pluralité d'impédances déterminées, associées chacune à un interrupteur dont l'ouverture et la fermeture sont commandées par le système de commande ;
- le dispositif médical est implantable dans le corps humain et est choisi parmi le groupe comprenant :
   - un tuteur vasculaire ou stent, au moins un capteur étant de préférence disposé sur une surface abluminale du tuteur vasculaire,
   - une valve cardiaque,
   - un stimulateur cardiaque,
   - un implant cochléaire,
   - un implant pour la gorge,
   - un implant orthopédique, ou
   - un tissu cellulaire ;
- chaque capteur est choisi parmi :
   - un capteur de cisaillement,
   - un capteur de pression,
   - un capteur d'impédance,
   - un capteur de dissipation de chaleur,
   - une jauge de contrainte, et
   - un capteur de débit, notamment du type à fil chaud ; et
- le dispositif médical implantable est un tuteur vasculaire avec au moins un capteur d'impédance disposé sur une surface abluminale du tuteur vasculaire.

L'invention se rapporte également à un système médical comprenant un dispositif médical tel que décrit ci-avant dans toutes ses combinaisons et une unité de réception d'informations depuis le dispositif médical, comprenant des moyens pour capter le champ électromagnétique émis par l'antenne du dispositif médical.

Le système médical peut comprendre en outre une unité d'interrogation du dispositif médical, de préférence confondue avec l'unité de réception d'information, comprenant de préférence des moyens pour émettre un champ électromagnétique adapté à créer un courant induit dans le circuit de mesure du dispositif médical.

Le système médical peut encore comprendre une unité de traitement des informations reçues par l'unité de réception, par exemple un ordinateur.

L'invention concerne également un procédé d'interrogation d'un dispositif médical tel que décrit ci-avant dans toutes ses combinaisons, notamment dans un système médical tel que décrit ci-avant dans toutes ses combinaisons, comprenant les étapes consistant à :
- alimenter le circuit de mesure du dispositif médical,
- activer le système de commande pour qu'il commande successivement l'ouverture ou la fermeture de chacun des interrupteurs, selon des configurations déterminées, et
- mesurer le champ électromagnétique émis par l'antenne du dispositif médical.

Les figures annexées feront bien comprendre comment l'invention peut être réalisée. Sur ces figures, des références identiques désignent des éléments semblables.

La figure 1 représente schématiquement un premier exemple de système médical comprenant un dispositif médical.

La figure 2 représente schématiquement un détail du circuit électrique du stent de la figure 1.

La figure 3 représente schématiquement un second exemple de système médical comprenant un dispositif médical.

La figure 4 représente schématiquement un troisième exemple de système médical comprenant un dispositif médical.

La figure 5 montre schématiquement un détail du dispositif médical de la figure 4.

La figure 6 représente schématiquement un quatrième exemple de système médical comprenant un dispositif médical.

Dans la suite de la description, les éléments identiques ou de fonction identique, portent le même signe de référence dans les différents modes de réalisation. À fin de concision de la présente description, ces éléments ne sont pas décrits en regard de chacun des modes de réalisation, seules les différences entre les modes de réalisation étant décrites.

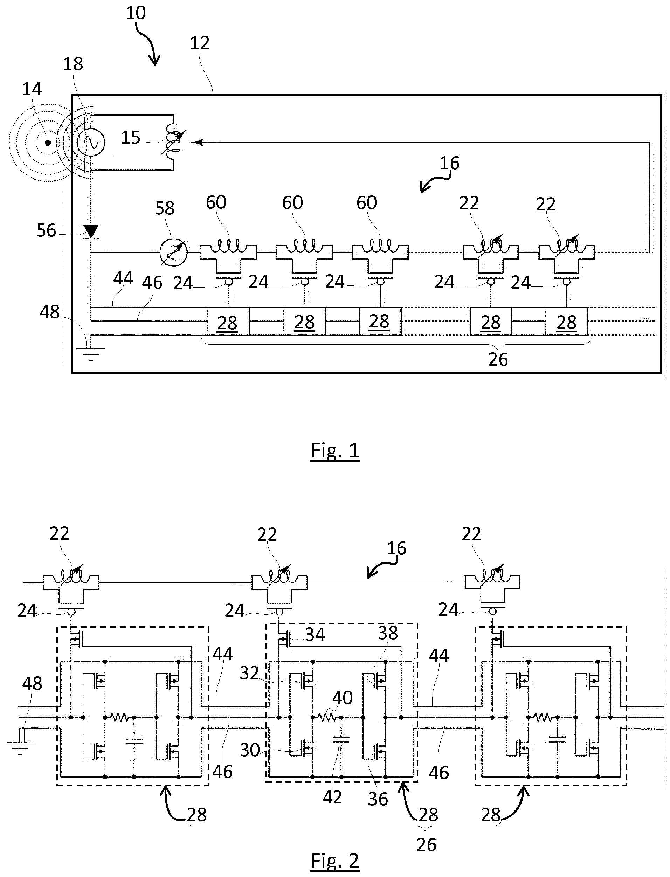

La figure 1 illustre schématiquement un système médical 10 comprenant un dispositif médical implantable 12 et une unité 14, ici unique, d'interrogation du dispositif médical 12 et de réception d'informations depuis ce même dispositif médical 12. Bien entendu, les unités d'interrogation et de réception d'informations peuvent, en variante, être distinctes. Le système médical 10 peut en outre comprendre une unité de traitement des informations reçues par l'unité de réception, par exemple un ordinateur.

Le dispositif médical implantable 12 comporte une impédance variable 15. La valeur de cette impédance variable 15 est commandée par une unité de commande non représentée, en fonction de l'impédance dans un circuit de mesure 16, reliant notamment les différents capteurs 22 du dispositif médical implantable. Le dispositif médical implantable 12 comporte encore une source d'énergie électrique, ici une source de courant électrique formée par le corps 18 du dispositif médical implantable 12. En effet, sous l'effet d'un champ électromagnétique émis par l'unité d'interrogation 14, le corps 18 du dispositif médical implantable 12 induit un courant. En variante, une antenne ou un induit séparé(e) et électriquement isolé(e) du corps 18 du dispositif médical implantable 12 peut également être prévu, notamment dans le cas où le dispositif médical implantable 12 n'est pas adapté, en tout ou partie, à avoir une fonction d'induit. Dans ce dernier cas notamment, une source d'énergie électrique pour le circuit de mesure peut comporter une surface conductrice de courant du dispositif médical implantable, adaptée à induire un courant électrique sous l'effet d'un champ électromagnétique. Une batterie ou pile électrique peut également être prévue comme source d'énergie électrique pour le dispositif médical implantable 12.

Le corps 18 du dispositif médical implantable 12 sert ici également d'antenne émettrice, pour émettre un champ électromagnétique vers l'extérieur du corps dans lequel le dispositif médical implantable est implanté. Par exemple, à intensité du courant induit constante de la source d'énergie électrique, l'intensité de ce champ dépend directement de l'impédance variable 15, fonction de l'impédance dans le circuit de mesure 16. Ainsi, l'intensité ou une norme du champ électromagnétique émis par le corps 18 du dispositif médical implantable 12 (ou plus généralement de l'antenne émettrice) est fonction de l'impédance du circuit de mesure 16. En variante, le dispositif médical implantable 12 peut comporter une antenne distincte du corps du dispositif médical implantable ou l'antenne peut être formée par une partie au moins du dispositif médical implantable.

Le dispositif médical implantable 12 est par exemple un stent. Le stent est un dispositif métallique tubulaire, de préférence maillé, glissé dans une cavité naturelle humaine (ou animale) pour la maintenir ouverte, comme décrit précédemment en introduction. Le stent peut par exemple être en alliage métallique ou en matériau polymère, mais d'autres matériaux sont également envisageables.

Le dispositif médical implantable 12 est muni de capteurs 22 à impédance variable en fonction de la grandeur physique qu'ils captent. Par grandeur physique, on entend ici toute propriété de la science de la nature qui peut être quantifiée par la mesure ou le calcul, et dont les différentes valeurs possibles s'expriment à l'aide d'un nombre réel quelconque ou d'un nombre complexe. Une grandeur physique inclut donc, par exemple, une longueur, un courant électrique, une tension, une impédance, une concentration en un élément chimique ou même la présence et/ou la concentration d'un élément biologique ou biochimique.

Les capteurs 22 sont répartis sur la surface du dispositif médical implantable. Dans le cas particulier du stent décrit ici, les capteurs 22 peuvent notamment être répartis :
- uniquement sur la surface « abluminale » du corps du stent, c'est-à-dire la surface opposée à la lumière à travers le stent, destinée à être en contact avec la paroi de la cavité à maintenir ouverte mais par sur la surface luminale ; ou
- uniquement sur la surface luminale mais pas sur la surface abluminale ; ou
- à la fois sur les surfaces luminale et abluminale ; et
- sur les surfaces reliant les surfaces luminale et abluminale.

Les capteurs peuvent être enduits d'un agent actif, par exemple pour limiter l'hyperplasie des tissus en contact avec le dispositif médical implantable, notamment quand ils sont positionnés sur la surface abluminale d'un stent ou plus généralement sur la surface extérieure d'un dispositif médical implantable destiné à être en contact avec la paroi de la cavité dans laquelle le dispositif médical est implantable.

Il est à noter que le positionnement d'un seul capteur, notamment d'un capteur de pression, sur la surface abluminale d'un stent, ou plus généralement sur la surface extérieure d'un dispositif médical implantable permet déjà d'avoir une information relative au mauvais positionnement du stent ou du dispositif médical implantable dans la cavité. Si la pression mesurée est faible (i.e. inférieure à une pression seuil), il est probable que le capteur n'est pas en contact avec une paroi de la cavité, mais plutôt avec du sang, par exemple. Dans le cas où deux capteurs ou plus sont disposés sur la surface abluminale ou extérieure, l'information peut être obtenue avec plus de précision en comparant les valeurs mesurées par les capteurs les unes avec les autres.

De préférence, les capteurs sont disposés aux emplacements du dispositif médical implantable, notamment d'un stent, subissant les déformations les moins importantes lors de la mise en place du dispositif médical implantable, ceci afin d'éviter d'endommager les capteurs.

Chacun des capteurs peut notamment être choisi parmi :
- un capteur de cisaillement,
- un capteur de pression,
- un capteur d'impédance,
- un capteur de dissipation de chaleur,
- une jauge de contrainte, et
- un capteur de débit du type « capteur à fil chaud » (de l'anglais « hot wire sensor »).

Les capteurs 22 sont des capteurs à impédance variable, c'est-à-dire des capteurs dont l'impédance varie en fonction de l'amplitude ou de l'intensité de la grandeur physique captée. Par suite, en cas de variation de l'amplitude de la grandeur physique captée par un capteur du dispositif médical implantable 12, l'impédance de ce capteur varie dans le circuit de mesure 16, de sorte que, en l'absence de toute autre variation dans le circuit de mesure 16, l'impédance du circuit de mesure 16 varie également.

Comme illustré, chaque capteur 22 est associé à un interrupteur 24 adapté à court-circuiter le capteur 22 auquel il est associé. Ici, ceci est réalisé en montant l'interrupteur 24 en dérivation (ou en parallèle) du capteur 22 auquel il est associé. Les capteurs 22 sont ici montés en série dans le circuit de mesure 16. Pour des raisons de facilité de réalisation et de miniaturisation, chaque interrupteur est ici réalisé par un transistor 24, en l'espèce un transistor MOS-FET au silicium, plus précisément un transistor MOS-FET à canal N, à enrichissement (ou n-MOS). Dans d'autres modes de réalisations, chaque interrupteur ou certains interrupteurs peuvent être réalisés par un autre type de transistor, notamment par un transistor FET, un transistor MOS-FET à appauvrissement, notamment un transistor MOS-FET à canal P à appauvrissement, par un MEMS (de l'anglais « Microelectromechanical system », ou par un interrupteur mécanique.

La figure 1 illustre en outre un système 26 de commande des interrupteurs 24, adapté à successivement commander l'ouverture ou la fermeture des interrupteurs 24 selon des configurations déterminées. Ici, le système de commande 26 comporte des modules de commande 28 disposés en série les uns des autres, chaque module de commande 28 étant adapté à commander l'ouverture ou la fermeture de l'interrupteur 24 auquel il est associé.

Dans le cas d'espèce, le système de commande 26 est conformé pour normalement maintenir fermés les interrupteurs 24 et pour les ouvrir successivement puis pour les refermer de sorte qu'à chaque instant un unique interrupteur 24 est ouvert.

Pour ce faire, chaque module de commande 28 est formé ici d'un circuit logique, réalisé au moyen de transistors 30, 32, 34, 36, 38, d'une résistance 40 et d'un condensateur 42. La résistance 40 et le condensateur 42 introduisent un temps de charge du condensateur 42 et un temps de décharge de ce même condensateur 42 dans le circuit logique. Pendant ces temps de charge et décharge, le module de commande 28 commande l'ouverture de l'interrupteur 24 associé. L'interrupteur 24 est maintenu fermé le reste de temps, court-circuitant alors le capteur 22 associé.

Plus précisément, et comme représenté sur la figure 2, ici, chaque module de commande 28 est réalisé au moyen de trois transistors 32, 34, 38 à canal P et deux transistors 30, 36 à canal N, comme suit (seules les liaisons ci-dessous sont réalisées) :
- des première et deuxième branches 44, 46 du circuit de mesure 16 sont branchées en parallèles,
- la grille du premier transistor 30 et la grille du deuxième transistor 32 sont reliées ensemble, ainsi qu'à la source du troisième transistor 34 et à la deuxième branche 46 du module de commande 28 précédant ;
- la grille du quatrième transistor 36 et la grille du cinquième transistor 38 sont reliées ensemble ainsi qu'à une borne de la résistance 40 et à une borne du condensateur 42 ;
- la source du premier transistor 30, la source du quatrième transistor 36 et une borne du condensateur 42 sont reliées à la masse 48 ;
- l'autre borne de la résistance 40, qui n'est pas reliée au condensateur 42, est reliée au drain du premier transistor 30 et au drain du deuxième transistor 32 ;
- le drain du quatrième transistor 36 et le drain du cinquième transistor 38 sont reliés ensemble à la deuxième branche 46 du module de commande 28 suivant, ainsi que la grille du troisième transistor 34 ;
- la source du deuxième transistor 32 et la source du cinquième transistor 38 sont reliés ensemble à la première branche 44 du module de commande 28 précédant ;
- le drain du troisième transistor 34 est relié à la grille du transistor 24 ayant une fonction d'interrupteur pour court-circuiter le capteur 22.

Avec un tel système de commande, la tension aux bornes du circuit de mesure 16, qui est égale à la somme des tensions aux bornes de chacun des capteurs montés en série dans le circuit de mesure, présente des pics successifs qui sont représentatifs de la tension aux bornes de chacun des capteurs. À chacun des pics successifs, représentatifs chacun de la tension aux bornes d'un capteur 22, correspond une intensité du champ électromagnétique émis par le corps 18 du dispositif médical implantable 12 ayant une fonction d'antenne émettrice.

Sur la figure 1, on constate la présence d'un redresseur 56 ainsi que d'un générateur de courant alternatif 58 dans le dispositif médical implantable 12. Ils permettent respectivement d'alimenter le circuit de commande 26 en courant continu et le circuit de mesure 16 avec un courant ayant une fréquence distincte de, notamment inférieure à, la fréquence du courant induit dans l'antenne 18. Ceci est peut être utile car la fréquence du courant induit est fonction du champ électromagnétique émis par l'unité 14, laquelle fréquence est de préférence choisie pour que l'onde électromagnétique soit peu absorbée par les tissus traversés. L'utilisation d'une telle fréquence dans le circuit de mesure pourrait nuire à la précision des mesures effectuées.

Le circuit de mesure 16 est par ailleurs complété sur la figure 1, par une combinaison d'ensembles d'une impédance 60, fixe et connue, et d'un interrupteur 24, commandé par un module de commande 28, comme cela est le cas pour les interrupteurs 24 associés aux capteurs 22. Cette combinaison d'impédances connues permet d'identifier le dispositif médical implantable interrogé, par exemple en associant une combinaison d'impédances 60 unique et connue à chaque dispositif médical implantable 12. Ceci est particulièrement utile dans le cas où plusieurs tels dispositifs médicaux implantables ont été implantés dans le corps d'un même patient. Certains pics de champ électromagnétique mesurés sont alors utilisés pour identifier le dispositif médical implantable 12, les autres pics pour déterminer les valeurs mesurées par chacun des capteurs du dispositif médical implantable 12 identifié. Par exemple, les premiers pics de champ électromagnétique mesurés peuvent être utilisés pour identifier le dispositif médical implantable 12 et les pics ultérieurs pour déterminer les valeurs mesurées par chacun des capteurs du dispositif médical implantable 12 identifié. En outre, ces impédances étant connues, elles permettent également de calibrer le système médical 10. En d'autres termes, ces impédances connues permettent de quantifier plus précisément les valeurs mesurées par les différents capteurs des différents dispositifs médicaux implantables.

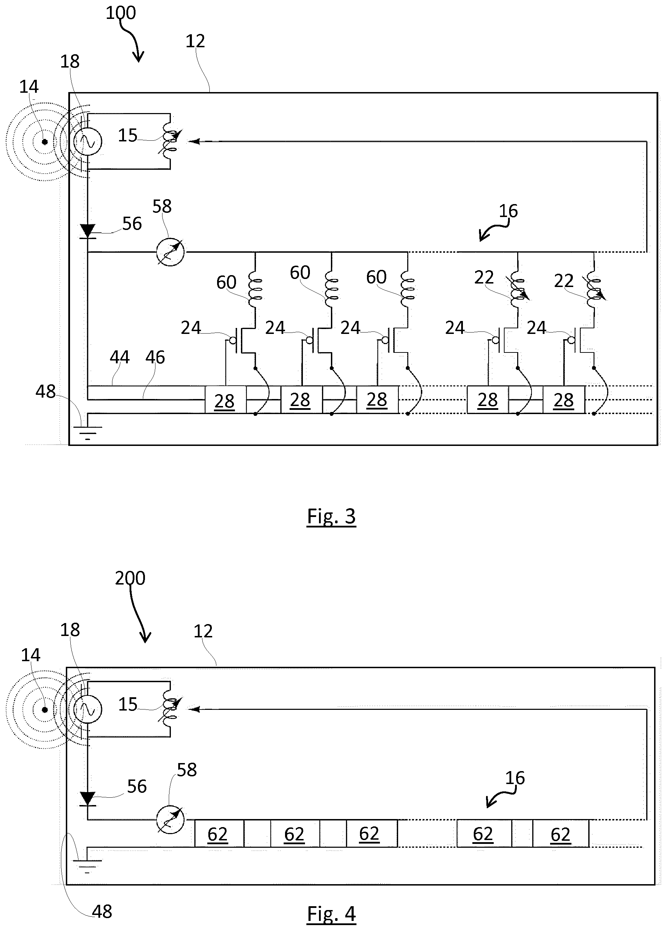

La figure 3 représente un deuxième exemple de système médical 100. Ce système médical est sensiblement identique à celui décrit précédemment. Cependant, dans ce mode de réalisation, dans le circuit de mesure 16 du dispositif médical implantable 12, les impédances 60 connues et les capteurs sont montés en série avec l'interrupteur 24 qui leur est associé, les ensembles formés d'une impédance 60 ou d'un capteur 22 et d'un interrupteur 24 étant montés en parallèle (ou en dérivation) les uns des autres. Par suite, les modules de commande 28 étant identiques à ceux décrits précédemment, le champ électromagnétique émis suite à la création d'un courant induit, correspond à la somme de toutes les impédances 60 et de tous les capteurs 22, moins un(e), chacune des impédances 60 et des capteurs 22 étant court-circuité(e) successivement.

En variante, bien entendu, on peut réaliser un module de commande 28 ayant un fonctionnement différent, qui commande la fermeture de l'interrupteur 24 durant un intervalle de temps seulement, l'interrupteur 24 étant ouvert le reste du temps. Un tel fonctionnement peut également être obtenu en conservant le module de commande 28 comme décrit précédemment et en remplaçant les transistors MOS-FET à enrichissement mis en œuvre en tant qu'interrupteurs 24 par des transistors MOS-FET à appauvrissement.

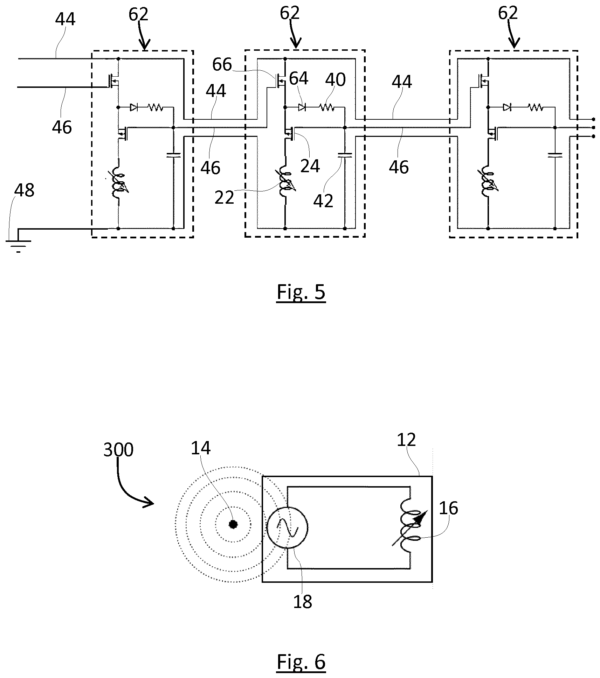

Les figures 4 et 5 illustrent un autre exemple de système médical 200. Selon cet exemple, la commande de l'interrupteur 24 de court-circuit du capteur 22 ou d'une impédance connue 60 est directement implantée dans un module 62 comprenant également l'impédance connue 60 ou le capteur 22, et l'interrupteur 24, ici réalisé par un transistor. Comme pour les autres exemples déjà décrits, une résistance 40 et un condensateur 42 sont mis en œuvre pour commander l'interrupteur 24 de telle sorte qu'il court-circuite l'impédance 60 ou le capteur 22 sauf durant un intervalle de temps de charge du condensateur 42.

Ici, comme représenté à la figure 5, chaque module 62 est réalisé comme suit :
- les première et deuxième branches 44, 46 sont en parallèles ;
- une borne du capteur 22 ou de l'impédance 60 est reliée à la masse 48 ;
- l'autre borne du capteur 22 ou de l'impédance 60 est reliée au drain du transistor 24 ;
- la grille du deuxième transistor 66 est reliée à la deuxième branche 46 du module 62 précédant ;
- le drain du deuxième transistor 66 est relié à la première branche 44 des modules 62 précédant et suivant ;
- la source du deuxième transistor 66 est reliée à la source du transistor 24 et à une diode 64 ;
- l'autre borne de la diode 64, qui n'est pas reliée aux transistors 24, 66, est relié à une borne d'une impédance 40 fixe ;
- l'autre borne de l'impédance 40, qui n'est pas reliée à la diode 64, est reliée à la grille du transistor 24, à une borne d'un condensateur 42, relié par son autre borne à la masse 48, et à la deuxième branche 46 du module 62 suivant.

Comme pour les exemples précédents, du fait de la configuration des modules 62, chaque capteur 22 et impédance 66 est successivement relié(e) à l'antenne 18 pour être alimenté(e), les autres capteurs 22 et impédances 66 étant pour leur part court-circuités.

Enfin, la figure 6 représente un quatrième exemple de mode de réalisation d'un système médical 300. Ce système médical 300 se distingue du précédent exemple 200, en ce que le circuit de mesure 16 est directement relié à l'antenne 18 pour l'émission d'un champ électromagnétique, sans l'intermédiaire d'une impédance variable distincte (le circuit de mesure 16 ayant lui-même une impédance variable) et d'une unité de commande de cette impédance variable en fonction de l'impédance du circuit de mesure 16. Le circuit électrique sur le dispositif médical 12 est alors particulièrement simplifié.

Bien entendu, on peut imaginer une structure où le circuit de mesure 16 est relié directement à l'antenne, le dispositif médical implantable comprenant également un circuit de commande associé à ce circuit de mesure et comme décrit par exemple en regard des figures 2 et 3.

En pratique, dans les modes de réalisation décrits précédemment, chaque module peut notamment être réalisé sous la forme suivante. Deux électrodes de mesures, par exemple de 60 x 60 µm², réalisées en un matériau conducteur électrique, par exemple en matériau polymère ou en alliage métallique, de préférence biocompatible, sont déposées sur un substrat polymérique biocompatible, isolant électrique (par exemple du parylène). Les composants électriques du système de commande et l'interrupteur sont implantés dans le substrat polymérique.

Les systèmes médicaux décrits précédemment permettent de mettre en œuvre un procédé d'interrogation du dispositif médical implantable 12.

Ce procédé comporte une première étape consistant à alimenter le circuit de mesure 16. De préférence, cette alimentation est réalisée par un courant induit dans une antenne ou dans le corps du dispositif médical implantable 12 quand celui-ci est conformé pour générer un courant induit. Ceci permet d'alimenter le circuit de mesure 16 uniquement quand une mesure est réalisée.

Le procédé se poursuit par une étape consistant à activer le système de commande du dispositif médical implantable pour qu'il commande successivement l'ouverture ou la fermeture de chacun des interrupteurs du dispositif médical implantable, selon des configurations déterminées. Il est à noter ici que dans le cadre des exemples décrits en regard des figures, cette activation est réalisée simultanément à l'alimentation du circuit de mesure 16, par induction, en réponse à l'émission d'un champ électromagnétique par le dispositif d'interrogation.

Le procédé se poursuit alors par une étape consistant à mesurer le champ électromagnétique émis par l'antenne du dispositif médical implantable. Cette mesure est réalisée durant un temps assez long pour que le système de commande ait pu commander un nombre assez importants de configurations différentes du circuit de mesure pour que la mesure permette de déterminer la valeur mesurée par chacun des capteurs 22 du dispositif médical implantable 12. Durant toute l'étape de mesure, l'antenne 14 émet de préférence un champ électromagnétique constant pour maintenir l'alimentation du circuit de mesure 16 et l'activation du système de commande 26.

De préférence, chaque configuration correspond au cas où tous les capteurs ou impédances du circuit de mesure sont court-circuités, sauf un(e). Ainsi, à partir du champ électromagnétique mesuré, il est possible de déterminer tout d'abord le dispositif médical implantable qui a répondu à l'interrogation. En effet, les premiers pics mesurés dans le champ électromagnétique émis par l'antenne correspondent à des impédances fixes, dont la combinaison permet d'identifier le dispositif médical implantable. Ces champs magnétiques mesurés peuvent également permettre de calibrer le système puisque les champs magnétiques mesurés correspondent à des impédances du circuit de mesure connues. Enfin, les champs magnétiques ultérieurs permettent de déterminer les valeurs mesurées par chacun des capteurs répartis sur le dispositif médical implantable.

Une unité de traitement peut être mise en œuvre pour déterminer la valeur mesurée par chaque capteur et le dispositif médical implantable qui a répondu à l'interrogation, notamment si les configurations commandées du circuit de mesure sont plus complexes.

Pour ce faire, l'unité de traitement peut notamment être adaptée à conduire des analyses de Fourrier des signaux mesurés de champs électromagnétiques émis par l'antenne du dispositif médical implantable, à comparer les signaux reçus (éventuellement traités) à des signaux mesurés précédemment et à en déduire les valeurs mesurées par les différents capteurs du dispositif médical implantable, une localisation pouvant être déterminée pour chacune des valeurs mesurées.

Il est à noter ici que le procédé décrit peut être mis en œuvre avec tout type de capteur à impédance variable en fonction de la grandeur physique qu'il détecte. Il est à noter également que les capteurs répartis sur le dispositif médical implantable peuvent être de natures différentes, c'est-à-dire qu'ils peuvent capter des grandeurs physiques différentes.

Le procédé décrit ci-avant peut notamment être mis en œuvre pour déterminer si le dispositif médical implantable est convenablement implanté (c'est-à-dire positionné) dans la cavité naturelle qu'il est censé maintenir ouverte, notamment, s'il est bien en contact avec la paroi de la cavité. En effet, l'effet d'un stent, par exemple mais cela est vrai pour la plupart des dispositifs médicaux implantables, est nettement réduit si celui n'est pas en appui sur la paroi de la cavité (notamment de la veine ou de l'artère) dans laquelle il est introduit.

Par exemple, en plaçant des capteurs de pression sur la surface abluminale du stent, c'est-à-dire sur la surface opposée à la lumière à travers le stent, celle qui est destinée à être en contact avec la paroi de la cavité dans laquelle le dispositif médical implantable est reçu. Le procédé décrit précédemment permet alors de déterminer si chacun de ces capteurs est en contact avec la paroi, puisqu'il permet de déterminer la pression mesurée par chacun des capteurs. Bien entendu, cette fonction de détermination de la position convenable du stent peut être combinée, c'est-à-dire que des capteurs, par exemple de pression, peuvent être disposés sur la surface abluminale du stent et des capteurs, éventuellement d'une autre grandeur physique, peuvent être disposés sur la surface luminale du stent.

En variante, des capteurs d'une même grandeur physique sont répartis sur la surface abluminale et sur la surface luminale, sensiblement à la même position sur le stent ou du dispositif médical implantable. En d'autres termes, des capteurs d'une même grandeur physique sont disposés au même point du stent, de part et d'autre du corps du stent. La comparaison des valeurs mesurées par chacun de ces couples de stent permet également d'avoir des indices d'une mauvaise position du stent dans la cavité. Notamment si le capteur sur la surface abluminale, qui devrait donc être en contact avec une paroi, mesure une valeur sensiblement identique au capteur sur la surface luminale, qui est en contact avec le sang, il est probable que le capteur sur la surface abluminale est en fait en contact avec du sang également, non avec une paroi. Il est donc probable que le stent est mal positionné dans la cavité.

Bien entendu, le procédé décrit précédemment peut permettre d'obtenir de nombreuses autres informations.

Notamment, il peut permettre de déterminer si un capteur disposé sur la surface luminale ou abluminale du stent ou, plus généralement, sur une surface d'un dispositif médical implantable, notamment sur une surface du dispositif médical implantable en contact avec une paroi de la cavité dans laquelle le dispositif médical est implanté ou sur une surface du dispositif médical implantable destinée à être en contact avec le sang, est ou non recouvert de tissu endothélial ou musculaire-lisse.

Il peut également permettre de déterminer la constitution du tissue recouvrant les capteurs répartis sur le dispositif médical implantable (notamment sur la surface luminale ou sur la surface abluminale d'un stent) par exemple par spectroscopie d'impédance électrique (EIS, de l'anglais « Electrical Impedance Spectroscopy »), notamment en appliquant des courants de fréquences distinctes dans le circuit de mesure.

L'invention ne se limite pas aux seuls exemples de réalisation décrit ci-avant en regard des figures, à titre d'exemples illustratifs et non limitatifs.

Notamment, le dispositif médical implantable peut être choisi parmi le groupe comprenant :
- une valve cardiaque,
- un stimulateur cardiaque,
- un implant cochléaire,
- un implant pour la gorge,
- un implant orthopédique, ou
- un tissu cellulaire (en anglais : « tissue-engineered construct »).

## Revendications

1. Dispositif médical (12) implantable dans le corps humain, ledit dispositif médical (12) comprenant un circuit électrique de mesure (16), dans lequel sont branchés au moins deux capteurs (22) à impédance variable en fonction d'une grandeur physique captée, une source d'énergie électrique (18) pour alimenter le circuit électrique de mesure (16), une antenne (18) pour émettre un champ électromagnétique fonction de l'impédance du circuit électrique de mesure (16), le dispositif médical étant **caractérisé en ce que** chacun des capteurs (22) est associé à un interrupteur (24) pour court-circuiter le capteur (22) dans ledit circuit de mesure (16), le dispositif médical (12) comprenant en outre un système (26) de commande des interrupteurs (24) pour successivement commander l'ouverture ou la fermeture des interrupteurs (24) les uns après les autres, selon des configurations déterminées et dans lequel le système (26) de commande des interrupteurs (24) comporte un circuit de commande (26), alimenté par la source d'énergie électrique (18), le système de commande (26) étant conformé pour normalement maintenir fermés les interrupteurs (24) et pour les ouvrir successivement puis pour les refermer de sorte qu'à chaque instant un unique interrupteur (24) est ouvert.

2. Dispositif médical selon la revendication 1, dans lequel chaque interrupteur (24) est formé par un ou des transistors, notamment un ou des transistors à effet de champs FET, plus particulièrement un ou des transistors à effet de champs à grille métal-oxyde MOS-FET à enrichissement ou à appauvrissement, à canal N ou à canal P, un ou des MEMS ou un ou des interrupteurs mécaniques.

3. Dispositif médical selon l'une quelconque des revendications 1 ou 2, dans lequel chaque ensemble d'un interrupteur (24) et d'un capteur (22) est monté en série et les ensembles d'un interrupteur (24) et d'un capteur (22) sont montés en parallèle les uns par rapport aux autres.

4. Dispositif médical l'une quelconque des revendications 1 ou 2, dans lequel chaque ensemble d'un interrupteur (24) et d'un capteur (22) est monté en parallèle et les ensembles d'un interrupteur (24) et d'un capteur (22) sont montés en série les uns des autres.

5. Dispositif médical selon l'une quelconque des revendications précédentes, dans lequel la source d'énergie électrique comporte une surface (18) conductrice de courant du dispositif médical implantable (12), adaptée à induire un courant électrique sous l'effet d'un champ électromagnétique.

6. Dispositif médical selon l'une quelconque des revendications précédentes, dans lequel au moins un des capteurs (22) est disposé sur une surface extérieure du dispositif médical (12), destinée à être en contact avec une partie du corps dans laquelle le dispositif médical est implantable.

7. Dispositif médical selon l'une quelconque des revendications précédentes, dans lequel l'antenne est formée par une partie au moins du dispositif médical.

8. Dispositif médical selon l'une quelconque des revendications précédentes, dans lequel le circuit de mesure comporte une pluralité d'impédances déterminées (60), associées chacune à un interrupteur (24) dont l'ouverture et la fermeture sont commandées par le système de commande (26).

9. Dispositif médical selon l'une quelconque des revendications précédentes, dans lequel le dispositif médical est choisi parmi le groupe comprenant : un tuteur vasculaire ou stent, au moins un capteur étant de préférence disposé sur une surface abluminale du tuteur vasculaire, une valve cardiaque, un stimulateur cardiaque, un implant cochléaire, un implant pour la gorge, un implant orthopédique, ou un tissu cellulaire.

10. Dispositif médical selon l'une quelconque des revendications précédentes, dans lequel chaque capteur est choisi parmi : un capteur de cisaillement, un capteur de pression, un capteur d'impédance, un capteur de dissipation de chaleur, une jauge de contrainte, et un capteur de débit, notamment du type à fil chaud.

11. Dispositif médical selon les revendications **9** et **10**, le dispositif médical implantable étant un tuteur vasculaire avec au moins un capteur d'impédance.

12. Système médical comprenant un dispositif médical (12) selon l'une quelconque des revendications précédentes et une unité de réception d'informations depuis le dispositif médical implantable, comprenant des moyens pour capter le champ électromagnétique émis par l'antenne du dispositif médical implantable (12).

13. Système selon la revendication 12**,** comprenant en outre une unité d'interrogation du dispositif médical, de préférence confondue avec l'unité de réception d'information, comprenant de préférence des moyens pour émettre un champ électromagnétique adapté à créer un courant induit dans le circuit de mesure du dispositif médical.

14. Système selon la revendication **12** ou **13**, comprenant en outre une unité de traitement des informations reçues par l'unité de réception, par exemple un ordinateur.

15. Procédé d'interrogation d'un dispositif médical selon l'une quelconque des revendications **1** à **11**, notamment dans un système médical selon l'une des revendications **12** à **14**, comprenant les étapes consistant à : alimenter le circuit de mesure du dispositif médical en énergie électrique, activer le système de commande pour qu'il commande successivement l'ouverture ou la fermeture de chacun des interrupteurs, selon des configurations déterminées, et mesurer le champ électromagnétique émis par l'antenne du dispositif médical.

## Patentansprüche

1. Medizinische Vorrichtung (12), die in den menschlichen Körper implantierbar ist, wobei die medizinische Vorrichtung (12) einen elektrischen Messkreis (16) umfasst, in den mindestens zwei Sensoren (22) mit in Abhängigkeit von einer gemessenen physikalischen Größe variabler Impedanz, eine Quelle elektrischer Energie (18) zur Versorgung des elektrischen Messkreises (16), eine Antenne (18), um ein elektromagnetisches Feld als Funktion der Impedanz des elektrischen Messkreises (16) zu senden, integriert sind, wobei die medizinische Vorrichtung **dadurch gekennzeichnet ist, dass** jeder der Sensoren (22) einem Schalter (24) zugeordnet ist, um den Sensor (22) in dem Messkreis (16) kurzzuschließen, wobei die medizinische Vorrichtung (12) ferner ein Steuersystem (26) der Schalter (24) umfasst, um nacheinander das Öffnen oder das Schließen der Schalter (24) hintereinander gemäß bestimmten Konfigurationen zu steuern und wobei das Steuersystem (26) der Schalter (24) einen Steuerkreis (26) aufweist, der von der Quelle elektrischer Energie (18) versorgt wird, wobei das Steuersystem (26) ausgebildet ist, um die Schalter (24) normalerweise geschlossen zu halten und um sie nacheinander zu öffnen, dann um sie wieder zu schließen, derart, dass zu jedem Moment ein einziger Schalter (24) geöffnet ist.

2. Medizinische Vorrichtung nach Anspruch 1, wobei jeder Schalter (24) von einem oder von Transistoren, insbesondere einem oder Transistoren mit Feldeffekt FET, spezieller einem oder Transistoren mit Feldeffekt und Metall-Oxid-Gate MOS-FET mit Enhancement oder mit Depletion, mit Kanal N oder mit Kanal P, einem oder MEMS oder einem oder mechanischen Schaltern gebildet ist.

3. Medizinische Vorrichtung nach einem der Ansprüche **1** oder **2**, wobei jede Anordnung aus einem Schalter (24) und einem Sensor (22) in Reihe angebracht ist und die Anordnungen aus einem Schalter (24) und einem Sensor (22) parallel zueinander angebracht sind.

4. Medizinische Vorrichtung nach einem der Ansprüche 1 oder 2, wobei jede Anordnung aus einem Schalter (24) und einem Sensor (22) parallel angebracht ist und die Anordnungen aus einem Schalter (24) und einem Sensor (22) in Reihe zueinander angebracht sind.

5. Medizinische Vorrichtung nach einem der vorangehenden Ansprüche, wobei die Quelle elektrischer Energie eine stromleitende Oberfläche (18) der implantierbaren medizinischen Vorrichtung (12) aufweist, die geeignet ist, unter der Wirkung eines elektromagnetischen Felds einen elektrischen Strom zu induzieren.

6. Medizinische Vorrichtung nach einem der vorangehenden Ansprüche, wobei mindestens einer der Sensoren (22) auf einer äußeren Oberfläche der medizinischen Vorrichtung (12) angeordnet ist, die bestimmt ist, mit einem Teil des Körpers in Kontakt zu sein, in den die medizinische Vorrichtung implantierbar ist.

7. Medizinische Vorrichtung nach einem der vorangehenden Ansprüche, wobei die Antenne von mindestens einem Teil der medizinischen Vorrichtung gebildet ist.

8. Medizinische Vorrichtung nach einem der vorangehenden Ansprüche, wobei der Messkreis eine Vielzahl bestimmter Impedanzen (60) aufweist, die jeweils einem Schalter (24) zugeordnet sind, dessen Öffnen und Schließen von dem Steuersystem (26) gesteuert wird.

9. Medizinische Vorrichtung nach einem der vorangehenden Ansprüche, wobei die medizinische Vorrichtung aus der Gruppe ausgewählt ist, die umfasst: eine Gefäßstütze oder Stent, wobei mindestens ein Sensor vorzugsweise auf einer abluminalen Oberfläche der Gefäßstütze angeordnet ist, eine Herzklappe, einen Herzschrittmacher, ein Cochlearimplantat, ein Halsimplantat, ein orthopädisches Implantat oder ein Zellgewebe.

10. Medizinische Vorrichtung nach einem der vorangehenden Ansprüche, wobei jeder Sensor ausgewählt ist aus: einem Scherkraftsensor, einem Drucksensor, einem Impedanzsensor, einem Wärmeableitungssensor, einem Dehnungsmesser und einem Durchflusssensor, insbesondere vom Typ mit Hitzdraht.

11. Medizinische Vorrichtung nach den Ansprüchen 9 und 10, wobei die implantierbare medizinische Vorrichtung eine Gefäßstütze mit mindestens einem Impedanzsensor ist.

12. Medizinisches System, umfassend eine medizinische Vorrichtung (12) nach einem der vorangehenden Ansprüche und eine Einheit zum Empfangen von Informationen von der implantierbaren medizinischen Vorrichtung, umfassend Mittel, um das von der Antenne der implantierbaren medizinischen Vorrichtung (12) gesendete elektromagnetische Signal aufzufangen.

13. System nach Anspruch **12**, umfassend ferner eine Einheit zum Abfragen der medizinischen Vorrichtung, die vorzugsweise mit der Einheit zum Empfangen von Informationen zusammenfällt, umfassend vorzugsweise Mittel, um ein elektromagnetisches Feld zu senden, das geeignet ist, einen in dem Messkreis der medizinischen Vorrichtung induzierten Strom zu erzeugen.

14. System nach Anspruch **12** oder **13**, umfassend ferner eine Einheit zum Empfangen der von der Empfangseinheit empfangenen Informationen, beispielsweise einen Rechner.

15. Verfahren zum Abfragen einer medizinischen Vorrichtung nach einem der Ansprüche **1** bis **11**, insbesondere in einem medizinischen System nach einem der Ansprüche **12** bis **14**, umfassend Schritte, die darin bestehen: Versorgen des Messkreises der medizinischen Vorrichtung mit elektrischer Energie, Aktivieren des Steuersystems, damit es nacheinander das Öffnen oder das Schließen von jedem der Schalter steuert, gemäß bestimmten Konfigurationen, und Messen des von der Antenne der medizinischen Vorrichtung gesendeten elektromagnetischen Felds.

## Claims

1. Medical device (12) implantable in a human body said medical device (12) comprising an electric measurement circuit (16), in which are connected at least two sensors (22) the impedance of which varies as a function of a sensed physical parameter, a source (18) of electrical power for powering the electric measurement circuit (16), an antenna (18) for emitting an electromagnetic field as a function of the impedance of the electric measurement circuit (16),
the medical device being **characterized in that**
each of the sensors (22) is associated with a switch (24) for short circuiting the sensor (22) in said measurement circuit (16), the medical device (12) further comprising a system (26) for controlling the switches (24) in order to successively command the opening or closing of the switches (24) one after another, according to determined configurations, and wherein the system (26) for controlling the switches (24) comprises a control circuit (26) powered by the electrical power source (18), the control system (26) being configured to normally keep the switches (24) closed and to open them successively then close them again so that only one single switch (24) is open at any time.

2. Medical device according to claim **1**, in which each switch (24) is formed of one or more transistors, notably one or more field effect transistors FETs, and, more particularly, one or more N-channel or P-channel enhancement or depletion MOSFET field effect transistors with metal oxide gate, one or more MEMSs or one or more mechanical switches.

3. Medical device according to any one of claims **1** or **2**, in which each assembly of a switch (24) and of a sensor (22) is mounted in series and the assemblies of a switch (24) and of a sensor (22) are mounted in parallel with one another.

4. Medical device according to any one of claim **1** or **2**, in which each assembly of a switch (24) and of a sensor (22) is mounted in parallel and the assemblies of a switch (24) and of a sensor (22) are mounted in series with one another.

5. Medical device according to any one of the preceding claims, in which the electrical power source comprises a current-conducting surface (18) of the implantable medical device (12) which is designed to induce an electric current under the effect of an electromagnetic field.

6. Medical device according to any one of the preceding claims, in which at least one of the sensors (22) is arranged on an exterior surface of the medical device (12), which surface is intended to be in contact with part of the body in which the medical device is implantable.

7. Medical device according to any one of the preceding claims, in which the antenna is formed by at least part of the medical device.

8. Medical device according to any one of the preceding claims, in which the measurement circuit comprises a plurality of determined impedances (60), each one associated with a switch (24) the opening and closing of which are commanded by the control system (26).

9. Medical device according to any one of the preceding claims, in which the medical device is chosen from the group comprising:
a vascular stent or stent, at least one sensor preferably being arranged on an abluminal surface of the vascular stent, a heart valve, a pacemaker, a cochlear implant, an implant for the throat, an orthopaedic implant, or a tissue-engineered construct.

10. Medical device according to any one of the preceding claims, in which each sensor is chosen from: a shear sensor, a pressure sensor, an impedance sensor, a heat dissipation sensor, a strain gauge, and a flow sensor, notably of the hot wire type.

11. Medical device according to claims **9** and **10**, the implantable medical device being a vascular stent with at least one impedance sensor.

12. Medical system comprising a medical device (12) according to any one of the preceding claims and a unit for receiving information from the implantable medical device, comprising means for sensing the electromagnetic field emitted by the antenna of the implantable medical device (12).

13. System according to claim **12**, further comprising a unit for interrogating the medical device, preferably coinciding with the information receiving unit, preferably comprising means for emitting an electromagnetic field able to create an induced current in the measurement circuit of the medical device.

14. System according to claim **12** or **13**, further comprising a data processing unit for processing the information received by the receiving unit, for example a computer.

15. Method for interrogating a medical device according to any one of claims **1** to **11**, notably in a medical system according to one of claims **12** to **14**, comprising the steps of: supplying electrical power to the measurement circuit of the medical device, activating the control system so that it successively commands the opening or closing of each of the switches in determined configurations, and measuring the electromagnetic field emitted by the antenna of the medical device.
